# EUROPEAN PATENT APPLICATION

(11) **EP 3 852 118 A1**
(43) Date of publication of application: **21.07.2021**
(21) Application number: 21151299.1
(22) Date of filing: 13.01.2021
(51) Int. Cl.: G16H 20/30, G16H 40/67

(54) **METHOD AND SYSTEM FOR OPERATING SMART MASSAGE SERVICE OF SMART MASSAGE CHAIR, AND COMPUTER-READABLE MEDIUM RECORDING THE METHOD**

(30) Priority: 17.01.2020 KR 20200006722
(71) Applicant: Bokjung Scale Corp., Seoul 04169 (KR)
(72) Inventor: LEE, Kun Young, Seoul 04169 (KR); HONG, Young Pyo, Goyang-si Gyeonggi-do 10527 (KR)
(74) Representative: Shearman, James Ward

(57) **Abstract**

Provided is a method of operating a smart massage service of a smart massage chair by using a computer system including at least one smart massage chair terminal, a user terminal, and a server computer connected through a network, including: receiving registration application information from the smart massage chair terminal or the user terminal; transmitting registration completion information to the smart massage chair terminal or the user terminal once a smart massage chair terminal of a company of interest or an authorized massage chair terminal of another company is confirmed by using the registration application information; receiving content request information from the registered smart massage chair terminal or the registered user terminal; and transmitting content information corresponding to the content request information to the smart massage chair terminal or the user terminal.

## Description

### CROSS-REFERENCE TO RELATED APPLICATION(S)

This application claims the benefit of and priority to Korean Patent Application No. 10-2020-0006722, filed on January 17, 2020, in the Korean Intellectual Property Office, the entire disclosure of which is incorporated herein by reference for all purposes.

### BACKGROUND

### 1. Field

The following description relates to a method and system for operating a smart massage service of a smart massage chair, and a computer-readable recording medium recording the method, and more particularly, to a method and system for operating a smart massage service of a smart massage chair, which allows users to enjoy various types of contents provided by content providers by networking the massage chair and to be provided with various smart massage services in connection with various subjects, such as an after-sales (AS) engineer, a customer center, an incident handler, and a remote operator, and a computer-readable recording medium recording the method.

### 2. Description of Related Art

In general, a massage is an act of stimulating the body by using a person's hand or a massage machine, and normalizing the function of the body, alleviating fatigue, or enhancing health through such stimulation of the body.

In recent years, mechanical massage devices have been developed to perform a massage, and among such massage devices, massage chairs that allow a user to be seated comfortably thereon and receive a full-body massage are widely used.

Since such conventional massage chairs cannot be connected to the outside through a network or the Internet, diverse types of software, coding information, content information, etc. that control the massage order or store various audio-visual information, once determined at the time of manufacturing the massage chair or purchasing the product, cannot be changed even if a user wants to modify the software or the information.

In addition, the conventional massage chairs cannot be linked with other companies' products, and hence there were problems in that the user cannot perform other tasks while receiving a massage.

Further, the conventional massage chairs cannot receive various smart massage services due to the inability to organically connect with other users, i.e., content providers who desire to create and provide various contents by themselves, after-sales (AS) engineers or customer centers that have to quickly perform after-sales service, incident handlers, such as on-premise incident handlers, off-premise incident handlers, 911 paramedics, ambulance operators, municipality officers, government officers, or the like, who have to be dispatched in case of emergency, and remote operators who can remotely control the massage chairs when a family member or a relative of the user receives the massage, or in case of after-sales service or emergencies.

Meanwhile, with the recent rapid development of wireless communication networks including a mobile communication network such as a second generation (2G), third generation (3G), fourth generation (4G), fifth generation (5G), or long term evolution (LTE) network, Wi-Fi communication network, Bluetooth communication network, cellular communication network, code division multiple access (CDMA) communication network, Ethernet communication network, WiMAX communication network, local area network (LAN), wide area network (WAN), radio frequency (RF) communication network, infrared communication network, and optical communication network, a variety of services are being developed using such wireless communication networks.

### SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

The present invention is devised to solve various problems including the problems discussed above, and is intended to provide a method and system for operating a smart massage service of a smart massage chair and a computer-readable recording medium recording the method, by which a massage chair terminal or a user terminal is connected to the outside through a network or the Internet to allow a user to control the massage order or to be provided with and enjoy various software, coding information, or content information in which a variety of audio-visual information is stored, the user or content providers can create or change and apply various contents at any time after the manufacture of a massage chair or after purchasing the product, and while the user is receiving a massage, the user can perform various other tasks, such as operating another company's product, by linking the massage chair with another company's product, and can also be provided with various smart massage services through organic connection with other users (i.e., contents provider who desire to create and provide a variety of contents by themselves, AS engineers or customer centers that have to quickly perform AS service, incident handlers, such as on-premise incident handlers, off-premise incident handlers, 911 paramedics, ambulance operators, municipality officers, government officers, or the like, who have to be dispatched in case of emergency, and remote operators who can remotely control the massage chairs when a family member or a relative of the user receives the massage, or in case of AS service or emergencies). However, these solutions are for illustrative purpose only, and the scope of the present invention is not limited thereto.

In one general aspect of the present invention, there is provided a method of operating a smart massage service of a smart massage chair by using a computer system including at least one smart massage chair terminal, a user terminal, and a server computer connected through a network, the method including: receiving registration application information from the smart massage chair terminal or the user terminal; transmitting registration completion information to the smart massage chair terminal or the user terminal once a smart massage chair terminal of a company of interest or an authorized massage chair terminal of another company is confirmed by using the registration application information; receiving content request information from the registered smart massage chair terminal or the registered user terminal; and transmitting content information corresponding to the content request information to the smart massage chair terminal or the user terminal.

In another general aspect of the present invention, there is provided a method of operating a smart massage service of a smart massage chair using a computer system including at least one smart massage chair terminal, a user terminal, and a server computer connected through a network, wherein the server computer may include a registration application information input program for receiving registration application information from the smart massage chair terminal or the user terminal, a registration program for transmitting registration completion information to the smart massage chair terminal or the user terminal once a smart massage chair terminal of a company of interest or an authorized massage chair terminal of another company is confirmed by using the registration application information, a content request information input program for receiving content request information from the registered smart massage chair terminal or the registered user terminal, a content information transmission program for transmitting content information corresponding to the content request information to the smart massage chair terminal or the user terminal, a registration application information database in which the registration application information is stored, and a content information database in which the content information is stored, and the method may include the steps of: (a) receiving the registration application information from the smart massage chair terminal or the user terminal through the registration application information input program; (b) transmitting the registration completion information to the smart massage chair terminal or the user terminal through the registration program once a smart massage chair terminal of a company of interest or an authorized massage chair terminal of another company is confirmed by using the registration application information; (c) receiving content request information from the registered smart massage chair terminal or the registered user terminal through the content request information input program; and (d) transmitting content information corresponding to the content request information to the smart massage chair terminal or the user terminal through the content information transmission program.

The registration application information may be formed by selecting at least one of model name information, unique information, and serial number information of a smart massage chair, user or purchaser's unique information, other company's massage chair information, input information of an installation technician, an after-sales (AS) engineer, or a customer center, barcode information, quick response (QR) code information, identification code information, radio frequency (RF) signal information, security information, authentication information, and combinations thereof.

The content information may be formed by selecting at least one of home appliance driving content information for driving another home appliance or an Internet of Things (IoT) product, home appliance state notification content information for informing a user of a state of another home appliance or an IoT product through a massage function, emergency notification content information for informing the user of an emergency situation through a massage function, tactile massage-based content information for allowing the user to enjoy sound or an image with a massage function, broadcast massage-based content information for allowing the user to enjoy TV broadcasting by associating a massage broadcast with a massage function, simultaneous massage content information for enabling a plurality of massage chairs to perform massage in the same pattern, and combinations thereof.

The content information may be massage-combined content information in which massage drive information for performing a massage by selecting at least one of the user's body parts (feet, legs, buttocks, back, hands, shoulders, neck, head, and the like), massage functions (massage start, massage end, massage type, massage intensity, massage speed, massage time, air and heat function, and the like), and combinations thereof is combined with audio-visual information formed by selecting at least one of voice information, sound information, music information, song information, autonomous sensory meridian response (ASMR) information, graphic information, photo information, image information, video information, movie information, broadcast program, augmented reality information, virtual reality information, tactile information, game information, and combinations thereof.

The massage-combined content information may be formed by selecting at least one of sound-instrument-matching content information in which a corresponding sound or a corresponding instrument sound is matched with each massaged body part of the user, image-effect-matching content information in which a corresponding image effect is matched with each massaged body part of the user, game-effect-matching content information in which a corresponding game effect or a corresponding game motion is matched with each massaged body part of the user, and combinations thereof.

The massage-combined content information may be content information associated with another home appliance, in which a corresponding operation of the another home appliance is matched with at least each massaged body part of the user so that the user can check an actual operation state of the another home appliance or an IoT product through the massaged body part, a speaker, or a monitor.

The massage-combined content information may be content information associated with another massage chair which includes driving command information of another massage chair to allow the user to remotely drive a massage part of the another massage chair.

The server computer may include an application information transmission program for transmitting application-related information including application installation information or update information to the smart massage chair terminal or the user terminal through the network or an application store and an application information database in which the application-related information is stored, and the method may further include the step of, prior to the step of (a), (e) transmitting the application-related information including application installation information or update information to the smart massage chair terminal or the user terminal through the network or the application store by using the application information transmission program.

The server computer may include a driving program of another company's product for transmitting driving content information of another company's product to a computer of another company that manufactures other home appliances or IoT products and a product driving content information in which the driving content information of another company's product is stored, and the method may further include the step of, after the step of (d), (f) transmitting, through the driving program of another company's product, the driving content information of another company's product to the computer of another company that manufactures other home appliances or IoT products.

The server computer may include a content information input program for receiving the content information from the content provider terminal or transmitting benefit information to the content provider terminal and the method may further include the step of, prior to the step of (c), (g) receiving the content information from the content provider terminal or transmitting the benefit information to the content provider terminal through the content information input program.

The server computer may include a content creation program information transmission program for transmitting content creation program information to the content provider terminal and a content creation program information database in which the content creation program information is stored, and the method may further include the step of, prior to the step of (g), (h) transmitting the content creation program information to the content provider terminal through the content creation program information transmission program.

The content creation program may include: an audio-visual information input program for inputting audio-visual information formed by selecting at least one of voice information, sound information, music information, song information, ASMR information, graphic information, photo information, image information, video information, movie information, a broadcast program, AR information, virtual reality information, tactile information, game information, and combinations thereof; a massage drive information input program for inputting the massage drive information for performing a massage by selecting at least one of the user's body parts (feet, legs, buttocks, back, hands, shoulders, neck, head, and the like), massage functions (massage start, massage end, massage type, massage intensity, massage speed, massage time, air and heat function, and the like), and combinations thereof corresponding to each characteristic of the audio-visual information; and a massage-combined content information storage program for storing the massage-combined content information in which the input audio-visual information is combined with the input massage drive information.

The server computer may include a failure handling program for receiving failure information from the smart massage chair terminal or the user terminal and transmitting the failure information to an AS engineer terminal and a failure information database in which the failure information is stored, and the method may further include the step of, after the step of (b), (i) receiving the failure information from the smart massage chair terminal or the user terminal and transmitting the failure information to the AS engineer terminal through the failure handling program.

The failure information may be formed by selecting at least one of failure location information indicating a part or location where a failure has occurred, failure code information indicating a failure code capable of identifying the type of failure, failure time information indicating a failure occurrence time, failure cause identification information indicating an operation status of a malfunctioning part before or after a certain period of time to enable identification of the cause of the failure, massage notification information for informing of a location of a malfunctioning part through a massage function in the event of a failure, failure history information indicating the failure history, and combinations thereof.

The server computer may include an incident handling program for receiving incident information from the smart massage chair terminal or the user terminal and transmitting the incident information to an incident handler terminal and an incident information database in which the incident information is stored, and the method may further include the step of, after the step of (b), (j) receiving incident information from the smart massage chair terminal or the user terminal and transmitting the incident information to the incident handler terminal through the incident handling program.

The server computer may include a remote control program for receiving remote control request information from the smart massage chair terminal or the user terminal and transmitting the remote control request information to a remote operator terminal and a remote control request information database in which the remote control request information is stored, and the method may further include the step of, after the step of (b), (k) receiving the remote control request information from the smart massage chair terminal or the user terminal and transmitting the remote control request information to the remote operator terminal through the remote operation program.

In still another general aspect of the present invention, there is provided a system for operating a smart massage chair and a smart massage service using a computer system including at least one smart massage chair terminal, a user terminal, and a server computer connected through a network, wherein the server computer may include a registration application information input program for receiving registration application information from the smart massage chair terminal or the user terminal, a registration program for transmitting registration completion information to the smart massage chair terminal or the user terminal once a smart massage chair terminal of a company of interest or an authorized massage chair terminal of another company is confirmed by using the registration application information, a content request information input program for receiving content request information from the registered smart massage chair terminal or the registered user terminal, a content information transmission program for transmitting content information corresponding to the content request information to the smart massage chair terminal or the user terminal, a registration application information database in which the registration application information is stored, and a content information database in which the content information is stored, and the server computer may include a control unit that is programmed to receive the registration application information from the smart massage chair terminal or the user terminal through the registration application information input program, transmit the registration completion information to the smart massage chair terminal or the user terminal through the registration program once a smart massage chair terminal of a company of interest or an authorized massage chair terminal of another company is confirmed by using the registration application information, receive content request information from the registered smart massage chair terminal or the registered user terminal through the content request information input program, and transmit content information corresponding to the content request information to the smart massage chair terminal or the user terminal through the content information transmission program.

In yet another general aspect of the present invention, there is provided a computer-readable recording medium having recorded thereon a method of operating a smart massage service of a smart massage chair using a computer system including at least one smart massage chair terminal, a user terminal, and a server computer connected through a network, wherein the server computer may include a registration application information input program for receiving registration application information from the smart massage chair terminal or the user terminal, a registration program for transmitting registration completion information to the smart massage chair terminal or the user terminal once a smart massage chair terminal of a company of interest or an authorized massage chair terminal of another company is confirmed by using the registration application information, a content request information input program for receiving content request information from the registered smart massage chair terminal or the registered user terminal, a content information transmission program for transmitting content information corresponding to the content request information to the smart massage chair terminal or the user terminal, a registration application information database in which the registration application information is stored, and a content information database in which the content information is stored, and the method may include the steps of: (a) receiving the registration application information from the smart massage chair terminal or the user terminal through the registration application information input program; (b) transmitting the registration completion information to the smart massage chair terminal or the user terminal through the registration program once a smart massage chair terminal of a company of interest or an authorized massage chair terminal of another company is confirmed by using the registration application information; (c) receiving content request information from the registered smart massage chair terminal or the registered user terminal through the content request information input program; and (d) transmitting content information corresponding to the content request information to the smart massage chair terminal or the user terminal through the content information transmission program.

Other features and aspects will be apparent from the following detailed description, the drawings, and the claims.

### BRIEF DESCRIPTION OF THE DRAWINGS

FIG. 1 is a conceptual diagram illustrating a system for operating a smart massage service of a smart massage chair according to some embodiments of the present invention.
FIG. 2 is a block diagram illustrating a content creation program of the system shown in FIG. 1 for operating a smart massage service of a smart massage chair.
FIG. 3 is a schematic diagram showing the relationships among an operator who operates a smart massage service of a smart massage chair of the present invention, a user, another company, a content provider, an AS engineer or a customer center, an incident handler, and a remote operator.
FIG. 4 is a conceptual diagram illustrating a method of operating a smart massage service of a smart massage chair according to some embodiments of the present invention.
FIG. 5 is a conceptual diagram illustrating a method of operating a smart massage service of a smart massage chair according to some other embodiments of the present invention.
FIG. 6 is a flowchart illustrating a method of operating a smart massage service of a smart massage chair according to still some other embodiments of the present invention.
FIGS. 7 and 8 are conceptual diagrams illustrating various examples of content information provided to a user by a method of operating a smart massage service of a smart massage chair according to some embodiments of the present invention.
FIGS. 9 to 15 are diagrams illustrating screens provided to terminals of the system illustrated in FIG. 1 for operating a smart massage service of a smart massage chair.

Throughout the drawings and the detailed description, unless otherwise described, the same drawing reference numerals will be understood to refer to the same elements, features, and structures. The relative size and depiction of these elements may be exaggerated for clarity, illustration, and convenience.

### [Explanation of reference numerals]

1: smart massage chair
10: smart massage chair terminal
20: user terminal
110: another company's computer
120: content provider terminal
130: AS engineer terminal
140: incident handler terminal
150: remote operator terminal
160: operator terminal
50: network
60: server computer
PG: program control unit
DB: database

### DETAILED DESCRIPTION

The invention is described more fully hereinafter with references to the accompanying drawings, in which exemplary embodiments of the invention are shown. This invention may, however, be embodied in many different forms and should not be construed as limited to the embodiments set forth herein. Rather, these exemplary embodiments are provided so that this disclosure is thorough, and will fully convey the scope of the invention to those skilled in the art. Throughout the drawings and the detailed description, unless otherwise described, the same drawing reference numerals are understood to refer to the same elements, features, and structures. The relative size and depiction of these elements may be exaggerated for clarity, illustration, and convenience.

The terms used in the present specification are merely used to describe particular embodiments, and are not intended to limit the present disclosure. As used herein, the singular forms "a," "an" and "the" are intended to include the plural forms as well, unless the context clearly indicates otherwise. It will be further understood that the terms "comprises" and/or "comprising," when used herein, specify the presence of stated features, integers, steps, operations, elements, and/or components, but do not preclude the presence or addition of one or more other features, integers, steps, operations, elements, components, and/or groups thereof.

Hereinafter, the embodiments of the present invention will be described with reference to the drawings which schematically illustrate the embodiments. In the drawings, for example, depending on a manufacturing technology and/or tolerance, modifications of illustrated shapes may be expected. Accordingly, it should be understood that the embodiments of the present disclosure are not limited to particular shapes in areas shown in the specification and may include, for example, changes in shape caused during a manufacturing process.

Hereinafter, a method and system for operating a smart massage service of a smart massage chair according to some embodiments of the present invention, and a computer-readable recording medium recording the method will be described in detail with reference to the drawings.

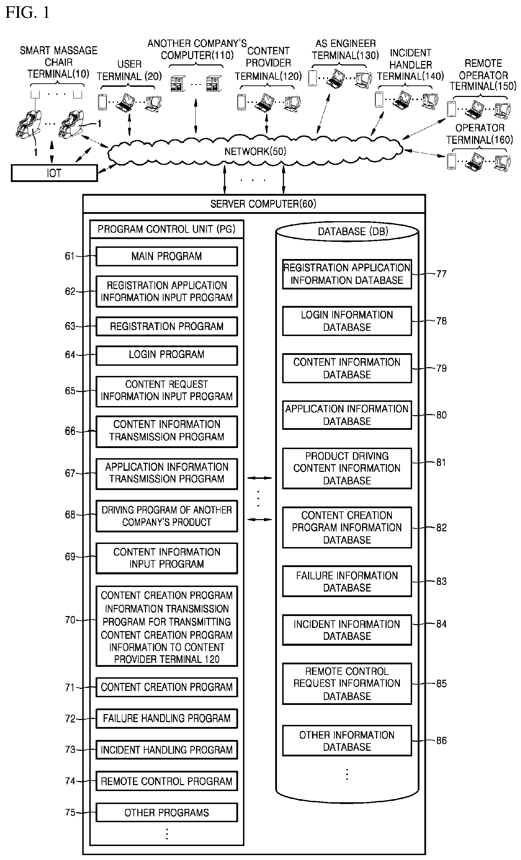

FIG. 1 is a conceptual diagram illustrating a system for operating a smart massage service of a smart massage chair according to some embodiments of the present invention.

First, as shown in FIG. 1, a system for operating a smart massage service of a smart massage chair according to some embodiments of the present invention may include a smart massage chair terminal 10 of a company of interest, a user terminal 20, another company's computer 110, a content provider terminal 120, an after-sales (AS) engineer terminal 130, an incident handler terminal 140, a remote operator terminal 150, an operator terminal 160, and a server computer 60 connected through a network 50.

Here, the network 50 may be connected to terminals of various electronic products, such as a robot cleaner, an air conditioner, a washing machine, and other electronic products including Internet of Things (IoT) or home automation products, in addition to the above-described terminals 10, 20, 110, 120, 130, 140, 150, and 160.

The server computer 60 may be programmed to receive registration application information from the smart massage chair terminal 10 or the user terminal 20; transmit registration completion information to the smart massage chair terminal 10 or the user terminal 20 once the smart massage chair terminal 10 of a company of interest or an authorized massage chair terminal of another company is confirmed using the registration application information; receive content request information from the registered smart massage chair terminal 10 or the registered user terminal 20; and transmit content information corresponding to the content request information to the smart massage chair terminal 10 or the user terminal 20, and may be a computer equipped in a company, a factory, a business entity, an organization, a data center, a head office, a branch office, a sales office, a business office, an agency, a massage chair manufacturer, or the like that can operate a smart massage service of the smart massage chair 1 through the network 50.

In addition, for example, the smart massage chair terminal 10 may be various terminals including various controllers, control panels, control pads, circuit boards, electronic devices, central processing units, computing devices, command input devices, remote controllers, keypads, keyboards, touch panels, storage devices, display devices, diagnostic devices, and the like that may be installed in the smart massage chair 1 or connected to the smart massage chair 1 by wired or wireless connection.

In addition, more specifically, for example, the smart massage chair terminal 10 may include a control panel, a remote control box, a smart pad, or the like that may be provided to a user when sold to the user by the manufacturer of the smart massage chair 1.

In addition, for example, the user terminal 20 is not only a terminal of a purchaser who has purchased the smart massage chair 1, but also a terminal of a user who can use the smart massage chair 1 or a terminal of a user who wants to receive various smart massage services or to offer the smart massage services to others.

More specifically, for example, the user terminal 20 may include all information terminal devices capable of processing various types of information. For example, the user terminals 20 may include not only various smartphones, but also various wearable devices, smart sensors, smart pads, mobile terminals, personal digital assistants (PDAs), notebook computers, laptop computers, smart cameras, smart camcorders, e-book readers, smart scanners, personal computers, and the like.

In particular, when a smartphone or a wearable device such as a smart watch or a smart band is used, the user's heart rate, blood pressure, blood oxygen content, moisture measurement, sleep pattern, step count, location information, and the like can be obtained in real-time. Also, user information, such as the user's psychological information and emotional state detection through facial expression analysis, which is more meaningful than existing information may be extracted and be used for the smart massage service.

In addition, for example, another company's computer 110 is a computer of another company that can manufacture or manage various IoT products that can be conveniently used while the user is receiving a massage, such as robot cleaners, televisions, air conditioners, washing machines, dryers, etc. Another company's computer 110 may be a computer equipped in a company, a factory, a business entity, an organization, a data center, a head office, a branch office, a business office, an agency, or a massage chair manufacturer that can cooperate with the smart massage service of the smart massage chair 1 through the network 50.

However, another company's computer 110 is not necessarily limited thereto, and another company's computer 110 may include all information terminal devices capable of processing various types of information, for example, various wearable devices, smart sensors, smart pads, mobile terminals, PDAs, notebook computers, laptop computers, smart cameras, smart camcorders, e-book readers, smart scanners, personal computers, and other server computers, as well as various types of smartphones.

Also, for example, the content provider terminal 120 may create various contents that can be provided in the smart massage service of the smart massage chair 1 through the network 50, and may include all terminals of the content provider who wishes to gain various benefits in return for providing the contents.

More specifically, for example, the content provider terminal 120 may include all information terminal devices capable of processing various types of information, for example, various wearable devices, smart sensors, smart pads, mobile terminals, PDAs, notebook computers, laptop computers, smart cameras, smart camcorders, e-book readers, smart scanners, personal computers, and other server computers, as well as various types of smartphones.

Meanwhile, for example, the smart massage chair terminal 10 and the user terminal 20 are not necessarily configured to be independent of each other, and for example, the smart massage chair terminal 10 and the user terminal 20 may be the same.

That is, the user may sufficiently control the smart massage chair with his or her own terminal without a separate remote controller, a control unit, a control panel, or a control pad provided at the time of purchase from the manufacturer.

Meanwhile, the user terminal 20 and the content provider terminal 120 are not necessarily configured to be independent of each other, and for example, the user terminal 20 and the content provider terminal 120 may be the same.

That is, while the user is provided with a smart massage service, the user may, if desired, create various contents and provide them to other users.

In addition, for example, the AS engineer terminal 130 may include all the terminals of an AS engineer, a customer center, or an installation technician capable of repairing the smart massage chair 1 through the network 50.

More specifically, for example, the AS engineer terminal 130 may include all information terminal devices capable of processing various types of information, for example, various wearable devices, smart sensors, smart pads, mobile terminals, PDAs, notebook computers, laptop computers, smart cameras, smart camcorders, e-book readers, smart scanners, personal computers, and other server computers, as well as various types of smartphones.

In addition, for example, the incident handler terminal 140 may include all the terminals of an accident handling crew, a paramedic, an emergency crew, an incident handler, an on-premise incident handler, an off-premise incident handler, a 911 paramedic, an ambulance operator, a municipality officer, and a government officer who are capable of handling various accidents that may occur in the smart massage chair 1 through the network 50.

More specifically, for example, the incident handler terminal 140 may include all information terminal devices capable of processing various types of information, for example, various wearable devices, smart sensors, smart pads, mobile terminals, PDAs, notebook computers, laptop computers, smart cameras, smart camcorders, e-book readers, smart scanners, personal computers, and other server computers, as well as various types of smartphones.

In addition, for example, the remote operator terminal 150 may be a terminal of a remote operator who is capable of remotely controlling the smart massage chair 1 when a family member or a relative of the user receives the massage, or in case of AS service or emergencies, and may include all information terminals capable of processing various types of information, for example, various wearable devices, smart sensors, smart pads, mobile terminals, PDAs, notebook computers, laptop computers, smart cameras, smart camcorders, e-book readers, smart scanners, personal computers, and other server computers, as well as various types of smartphones.

In addition, for example, the operator terminal 160 is a terminal or a computer of an individual, a company, a factory, a business entity, a central control center, a head office, a branch office, a sales office, or a computer manager that manages and operates the server computer 60. The operator terminal 160 is not necessarily limited to a computer or a smartphone, and may include various information terminals, wearable terminals, PDAs, smart watches, smart pads, cameras, camcorders, notebook computers, laptop computers, e-book readers, personal computers, and other server computers that can receive various text information, number information, or image information, and select various commands.

Meanwhile, for example, the operator terminal 160 is not necessarily provided independently of the AS engineer terminal 130, the incident handler terminal 140, and the remote operator terminal 150. For example, the operator terminal 160, the AS engineer terminal 130, the incident handler terminal 140, and the remote operator terminal 150 may be the same.

In other words, the operator may be an AS engineer, a customer center, an incident handler, or a remote operator to provide a smart massage service to users.

For example, the smart massage chair terminal 10 of FIG. 1, the user terminal 20, another company's computer 110, the content provider terminal 120, the AS engineer terminal 130, the incident handler terminal 140, the remote operator terminal 150, and the operator terminal 160 are installed with various applications, hybrid applications, programs, etc., and are connected to one another through the network 50. The terminals connected through the network 50 may use a mobile communication network, such as conventional 2G, 3G, 4G, 5G, and LTE networks, and the like, and a communication network, such as Wi-Fi communication network, Bluetooth communication network, cellular communication networks, CDMA communication networks, LTE communication network, Ethernet communication network, WiMAX communication network, LAN, WAN, RF communication network, infrared communication network, optical communication network, and the like, and may have an Internet browser (e.g., Netscape, Internet Explorer, etc.) that can display web contents in the form of HTML, XML, HTML5, or the like, or a protocol device for accessing an on-premise, off-premise, short-range, or long-range wired/wireless network.

Meanwhile, as shown in FIG. 1, the server computer 60 may include a program control unit PG for controlling programs and a database DB for storing various types of information.

In particular, the program control unit PG, as shown in FIG. 1, may include a main program 61 for operating the whole programs, a registration application information input program 62 for receiving the registration application information from the smart massage chair terminal 10 or the user terminal 20, a registration program 63 for transmitting registration completion information to the smart massage chair terminal 10 or the user terminal 20 once the smart massage chair terminal 10 of a company of interest or an authorized massage chair terminal of another company is confirmed by using the registration application information, a login program 64 for receiving log information from various terminals, a content request information input program 65 for receiving content request information from the registered smart massage chair terminal 10 or the registered user terminal 20, a content information transmission program 66 for transmitting content information corresponding to the content request information to the smart massage chair terminal 10 or the user terminal 20, an application information transmission program 67 for transmitting application-related information including application installation information or update information to the smart massage chair terminal 10 or the user terminal 20 through the network 50 or an application store, a driving program 68 of another company's product for transmitting driving content information of another company's product to the computer 110 of another company that manufactures other home appliances or IoT products, a content information input program 69 for receiving the content information from the content provider terminal 120 or transmitting benefit information to the content provider terminal 120, a content creation program information transmission program 70 for transmitting content creation program information to the content provider terminal 120, a content creation program 71 for generating massage-combined content information by combining audio-visual information and massage drive information, a failure handling program 72 for receiving failure information from the smart massage chair terminal 10 or the user terminal 20 and transmitting the failure information to the AS engineer terminal 130, an incident handling program 73 for receiving incident information from the smart massage chair terminal 10 or the user terminal 20 and transmitting the incident information to the incident handler terminal 140, a remote control program 74 for receiving remote control request information from the smart massage chair terminal 10 or the user terminal 20 and transmitting the remote control request information to the remote operator terminal 150, and other programs for performing functions, such as various graphics, payment, a bulletin board, and the like.

Here, for example, the main program 61, which operates the whole programs, may be expressed online in the form of a main screen of a worker management homepage, an application, or a program and may be a program capable of controlling all the aforementioned programs by receiving various information and command signals from the smart massage chair terminal 10, the user terminal 20, or the operator terminal 160.

In addition, for example, the registration application information input program 62 is a program for receiving the registration application information from the smart massage chair terminal 10, the user terminal 20, another company's computer 110, the content provider terminal 120, the AS engineer terminal 130, the incident handler terminal 140, the remote operator terminal 150, and the operator terminal 160, and may be a program capable of receiving unique information from the above-described terminals, allowing them to agree to standard terms and conditions or terms and conditions for information collection and use, and receiving real name verification, public IPIN, ID, password, email, mobile phone number, address, personal information, patient information, social security number information, unique number, phone numbers stored in a SIM card, and the like.

In addition, for example, the registration program 63 may be a program that uses the registration application information to confirm that other registration conditions, such as the smart massage chair terminal 10 of a company of interest or an authorized massage chair terminal of another company, are satisfied and transmits the registration completion information to the smart massage chair terminal 10 or the user terminal, wherein the registration conditions may be input in advance to the server computer 60.

More specifically, for example, the registration application information, which is the criterion of the above-described registration conditions, may be formed by selecting at least one of model name information, unique information, and serial number information of a smart massage chair, user or purchaser's unique information, other company's massage chair information, input information of an installation technician, a AS engineer, or a customer center, barcode information, quick response (QR) code information, identification code information, radio frequency (RF) signal information, security information, authentication information, and combinations thereof.

Also, for example, the login program 64, which is a program capable of receiving login information from the smart massage chair terminal 10, the user terminal 20, another company's computer 110, the content provider terminal 120, the AS engineer terminal 130, the incident handler terminal 140, the remote operator terminal 150, and the operator terminal 160 and performing a login process, may be a program that allows the user, another company's personnel in charge, the AS engineer, the customer center, the incident handler, the remote operator, or the operator to access the server computer 60 and then log in by automatically or manually entering ID, password, various identification symbols, email address, phone number, or the like.

In addition, for example, the content request information input program 65 is a program for receiving the content request information from the registered smart massage chair terminal 10 or the registered user terminal 20, through which the user can request desired content information.

Here, for example, the content information that users may be formed by selecting at least one of home appliance driving content information for driving another home appliance or an IoT product, home appliance state notification content information for informing the user of a state of another home appliance or an IoT product through a massage function, emergency notification content information for informing the user of an emergency situation through a massage function, tactile massage-based content information for allowing the user to enjoy sound or an image with a massage function, broadcast massage-based content information for allowing the user to enjoy TV broadcasting by associating a massage broadcast with a massage function, simultaneous massage content information for enabling a plurality of massage chairs to perform massage in the same pattern, and combinations thereof.

Therefore, the user may not only drive other home appliances, such as the IoT product, by using the home appliance driving content information, but also allow a massage function, such as tapping or kneading, to be performed by using the home appliance notification content information so that the user can be informed, for example, when a washing machine finishes washing or cooking with a gas stove, a microwave oven, an electric oven, or the like is finished.

In addition, even if the user falls asleep in the massage chair, the massage function, such as tapping or kneading, may be performed by using the emergency notification content information so that the user can be informed when an emergency situation is detected using a fire detector, a light detector, a smoke detector, or the like.

By using the tactile massage-based content information or the broadcast massage-based content information, it is possible to allow the user's body to feel the sound of various videos, music, commercial songs, and the like through the sense of touch transmitted to the body during the massage.

In addition, by using the simultaneous massage content information, it is possible to allow multiple users to enjoy various services, such as receiving a massage in the same pattern at the same time through separate programs or broadcasting programs.

Here, for example, the content information that users can select may be massage-combined content information in which massage drive information for performing a massage by selecting at least one of the user's body parts (feet, legs, buttocks, back, hands, shoulders, neck, head, and the like), massage functions (massage start, massage end, massage type, massage intensity, massage speed, massage time, air and heat function, and the like), and combinations thereof is combined with audio-visual information formed by selecting at least one of voice information, sound information, music information, song information, autonomous sensory meridian response (ASMR) information, graphic information, photo information, image information, video information, movie information, broadcast program, augmented reality (AR) information, virtual reality information, tactile information, game information, and combinations thereof.

More specifically, for example, the massage-combined content information may be formed by selecting at least one of sound-instrument-matching content information in which a corresponding sound or a corresponding instrument sound is matched with each massaged body part of the user, image-effect-matching content information in which a corresponding image effect is matched with each massaged body part of the user, game-effect-matching content information in which a corresponding game effect or a corresponding game motion is matched with each massaged body part of the user, and combinations thereof.

In addition, for example, the content information transmission program 66 is a program for transmitting content information corresponding to the content request information to the smart massage chair terminal 10 or the user terminal 20, through which the operator may transmit content information desired by the user.

Accordingly, the user may receive a massage service, which may be tedious, with fun and joy while enjoying various audio-visual information through the massage-coupled content information.

In addition, for example, the application information transmission program 67 is a program for transmitting application-related information including application installation information or update information to the smart massage chair terminal 10 or the user terminal 20 through the network 50 or an application store. Through the application information transmission program 67, the user may supplement or improve the function of the smart massage chair 1 by installing, upgrading, or updating a new latest program and the massage chair may always feel fresh as if newly purchased and make the user feel as if management is consistently provided.

In addition, for example, the driving program 68 of another company's product is a program for transmitting content information for driving another company's product to the computer 110 of another company that manufactures other home appliances or IoT products, and more specifically, for example, the driving program 68 of another company's product may be a kind of massage-combined content information, which is content information associated with another home appliance, in which a corresponding operation of the another home appliance is matched with at least each massaged body part of the user so that the user can check an actual operation state of the another home appliance or an IoT product through the massaged body part, a speaker, or a monitor.

In addition, for example, the content information input program 69 is a program for receiving the content information from the content provider terminal 120 or transmitting benefit information to the content provider terminal 120. The content creation program information transmission program 70 is a program for transmitting content creation program information to the content provider terminal 120, through which the operator may provide a variety of content in various forms to the user through the multiple content providers without needing to directly providing content, and in return, may offer various benefits, such as a content provision fee, a product discount coupon, a free use coupon, a tribute to the Hall of Fame, or the like, to the content providers.

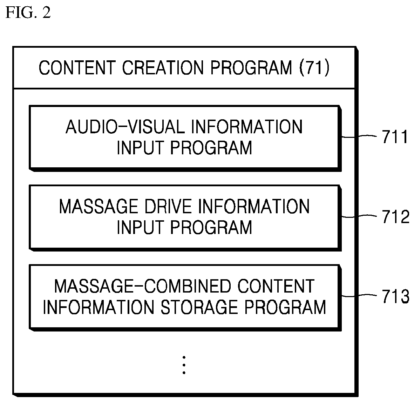

FIG. 2 is a block diagram illustrating a content creation program of the system shown in FIG. 1 for operating a smart massage service of a smart massage chair.

As shown in FIGS. 1 and 2, for example, the content creation program 71 is a program for creating massage-combined content information by combining audio-visual information and massage drive information. More specifically, for example, the content creation program 71 may include an audio-visual information input program 711, a massage drive information input program 712, and a massage-combined content information storage program 713. The audio-visual information input program 711 is provided for inputting audio-visual information formed by selecting at least one of voice information, sound information, music information, song information, ASMR information, graphic information, photo information, image information, video information, movie information, a broadcast program, AR information, virtual reality information, tactile information, game information, and combinations thereof. The massage drive information input program 712 is provided for inputting the massage drive information for performing a massage by selecting at least one of the user's body parts (feet, legs, buttocks, back, hands, shoulders, neck, head, and the like), massage functions (massage start, massage end, massage type, massage intensity, massage speed, massage time, air and heat function, and the like), and combinations thereof corresponding to each characteristic of the audio-visual information. The massage-combined content information storage program 713 is provided for storing the massage-combined content information in which the input audio-visual information is combined with the input massage drive information.

Accordingly, the content provider may use online or download the content creation program 71 provided in the server computer 60 of the present invention to store the massage-combined content information in which the input audio-visual information is combined with the input massage drive information, and may upload the massage-combined content information to the server computer 60, thereby providing the massage-combined content information for use by multiple users.

Therefore, the operator may activate the content market in which the users or the content providers can provide content voluntarily, thereby allowing consumers to create a new high-value added market like a game, as well as creating new fun and promoting passion, which leads to further improvement of customer loyalty.

Meanwhile, as shown in FIG. 1, for example, the failure handling program 72 is a program for receiving failure information from the smart massage chair terminal 10 or the user terminal 20, through which, in the event of a failure in the massage chair 1, an AS engineer or a customer center may be automatically and promptly called by an automatic diagnostic circuit or an automatic diagnostic logic installed in the smart massage chair 1 or the smart massage chair terminal 10, or the user may promptly call the AS engineer or the customer center using the smart massage chair terminal 10 or the user terminal 20.

Here, the above-described automatic diagnostic circuit or automatic diagnostic logic may include various sensors, diagnostic circuits, or diagnostic programs capable of determining a malfunction state by comparing a state signal during normal operation and a state signal during abnormal operation.

More specifically, for example, the failure information may be formed by selecting at least one of failure location information indicating a part or location where a failure has occurred, failure code information indicating a failure code capable of identifying the type of failure, failure time information indicating a failure occurrence time, failure cause identification information indicating an operation status of a malfunctioning part before or after a certain period of time to enable identification of the cause of the failure, massage notification information for informing of a location of a malfunctioning part through a massage function in the event of a failure, failure history information indicating the failure history, and combinations thereof.

Accordingly, the operator, the AS engineer, or a repair center can identify not only the location of the malfunctioning part of the smart massage chair 1 or the type of the malfunction, but also the cause of the failure through information or history before the failure occurs, and may inform the user of the state of the failure through the massage function, such as tapping or kneading.

In addition, for example, the incident handling program 73 is a program for receiving incident information from the smart massage chair terminal 10 or the user terminal 20 and transmitting the incident information to the incident handler terminal 140, through which, when various incidents, such as a child or pet being caught in the smart massage chair 1, too strong massage, the user's feeling of pain or unwell, or the like, occur, an incident handler is quickly called by using an accident emergency switch or an incident report command input device installed in the smart massage chair 1 or the smart massage chair terminal 10, or the user may quickly call an incident handler by using the smart massage chair terminal 10 or the user terminal 20.

Here, the above-described accident emergency switch or incident report command input device may include various sensors, incident detection circuits, incident determination programs capable of determining various safety incidents such as when different safety switches are activated or abnormally activated, high-pressure operation continues for a certain period of time or longer, a camera captures an image showing a child or a pet being caught in the smart massage chair, or a scream is detected through a microphone.

In addition, for example, the remote control program 74 is a program for receiving remote control request information from the smart massage chair terminal 10 or the user terminal 20 and transmitting the remote control request information to the remote operator terminal 150. More specifically, the remote control request information may include, for example, emergency control request information in case of a failure or incident, as well as content information associated with another massage chair which includes driving command information of another massage chair to allow the user to remotely drive a massage part of the another massage chair.

Therefore, the smart massage chair terminal 10 or the user terminal 20 may be connected to the outside through a network or the Internet to allow the user to control the massage order or to be provided with and enjoy various software, coding information, or content information in which a variety of audio-visual information is stored. In addition, users or content providers may create or change and apply various contents at any time after the manufacture of the massage chair or after purchasing the product, and while a user is receiving a massage, the user may perform various other tasks, such as operating another company's product, by linking the massage chair with another company's product, and may also be provided with various smart massage services through organic connection with other users (i.e., content providers who desire to create and provide a variety of contents by themselves, AS engineers or customer centers that have to quickly perform AS service, incident handlers, such as on-premise incident handlers, off-premise incident handlers, 911 paramedics, ambulance operators, municipality officers, government officers, or the like, who have to be dispatched in case of emergency, and remote operators who can remotely control the massage chairs when a family member or a relative of the user receives the massage, or in case of AS service or emergencies), through which the smart massage chair 1 can be smoothly operated.

Here, the above-described programs may be operated in association with an executable program, screen control program, or user application downloaded or installed in various terminals, such as the smart massage chair terminal 10, the user terminal 20, other company's computer 110, the content provider terminal 120, the AS engineer terminal 130, the incident handler terminal 140, the remote operator terminal 150, and the operator terminal 160.

However, the above-described programs are not necessarily limited to being associated with an executable program or a smart phone application, and may be associated with all various types of terminals.

Meanwhile, as shown in FIG. 1, the database DB may include a registration application information database 77 in which the registration application information is stored, a login information database 78 in which the login information is stored, a content information database 79 in which the content information is stored, an application information database 80 in which the application-related information is stored, a product driving content information database 81 in which the driving content information of another company's product is stored, a content creation program information database 82 in which the content creation program information is stored, a failure information database 83 in which the failure information is stored, an incident information database 84 in which the incident information is stored, and a remote control request information database 85 in which the remote control request information is stored, an other information database 86 in which other information, such as worker management homepage, various advertisements, promotions, payment, a bulletin board, and the like, is stored.

Accordingly, the server computer 60 may receive the registration application information from the smart massage chair terminal 10 or the user terminal 20 through the registration application information input program 62, transmit the registration completion information to the smart massage chair terminal 10 or the user terminal through the registration program 63 once the smart massage chair terminal 10 of a company of interest or an authorized massage chair terminal of another company is confirmed by using the registration application information, receive the content request information from the registered smart massage chair terminal 10 or the registered user terminal 20 through the content request information input program 65, and perform a smart massage service of the smart massage chair to transmit the content information corresponding to the content request information to the smart massage chair terminal 10 or the user terminal 20.

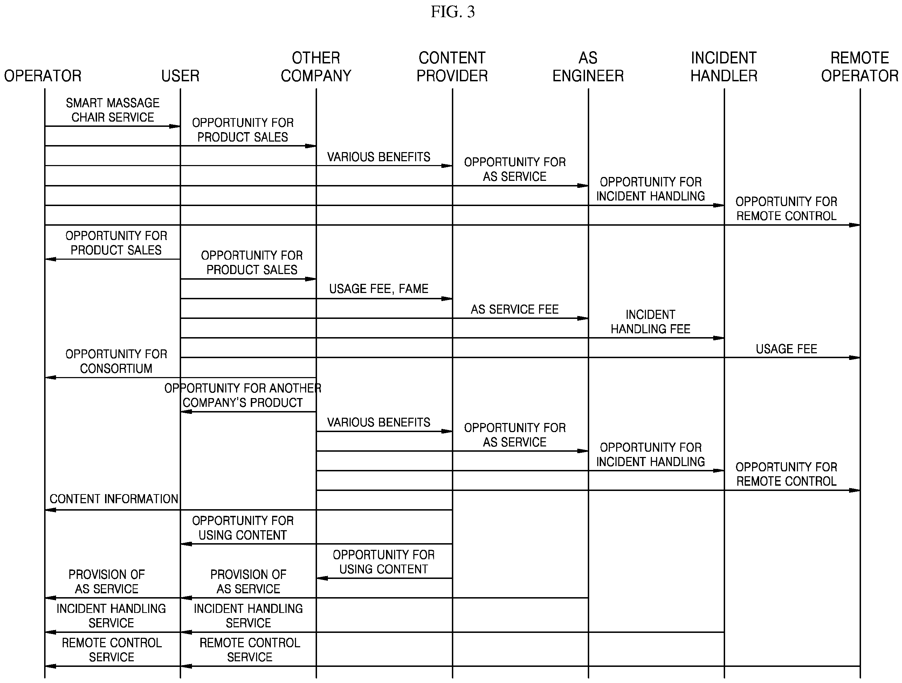

FIG. 3 is a schematic diagram showing the relationships among an operator who operates a smart massage service of a smart massage chair of the present invention, a user, another company, a content provider, an AS engineer or a customer center, an incident handler, and a remote operator.

As shown in FIG. 3, the operator may provide a smart massage chair service to the user, give another company or a manufacturer of another's company's product an opportunity for product sales, offer various benefits to content providers in exchange for providing contents, and provide a job opportunity to the AS engineer, the customer center, the incident handler, or the remote operator. In addition, the user may give the operator or another company an opportunity for product sales, pay a usage fee and give fame to the content provider, and also provide a job opportunity to the AS engineer, the customer center, the incident handler, or the remote operator, so that a useful business model (profit-generating model) or computer management model that can be beneficial to all involved can be provided.

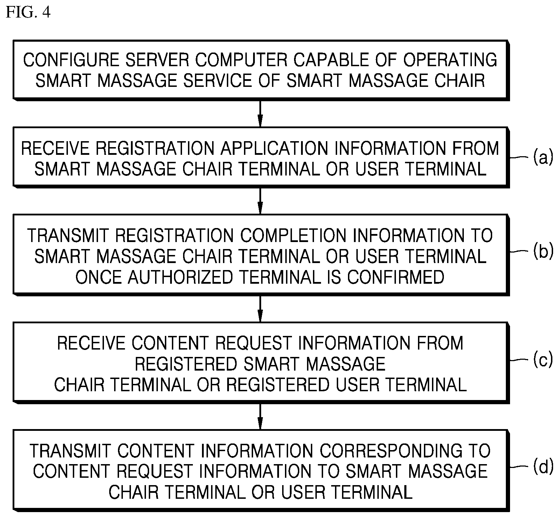

FIG. 4 is a conceptual diagram illustrating a method of operating a smart massage service of a smart massage chair according to some embodiments of the present invention.

As shown in FIGS. 1 to 4, when sequentially showing the method of operating a smart massage service of a smart massage chair according to some embodiments of the present invention, first, the server computer 60 capable of providing a smart massage service of a smart massage chair is configured and the method includes the steps of: (a) receiving registration application information from the smart massage chair terminal 10 or the user terminal 20 through the registration application information input program 62; (b) transmitting registration completion information to the smart massage chair terminal 10 or the user terminal 20 through the registration program 63 once the smart massage chair terminal 10 of the company of interest or an authorized massage chair terminal of another company is confirmed by using the registration application information; (c) receiving content request information from the registered smart massage chair terminal 10 or the registered user terminal 20 through the content request information input program 65; and (d) transmitting content information corresponding to the content request information to the smart massage chair terminal 10 or the user terminal 20 through the content information transmission program 66.

However, the present invention is not necessarily limited to the drawings, and various steps may be additionally included.

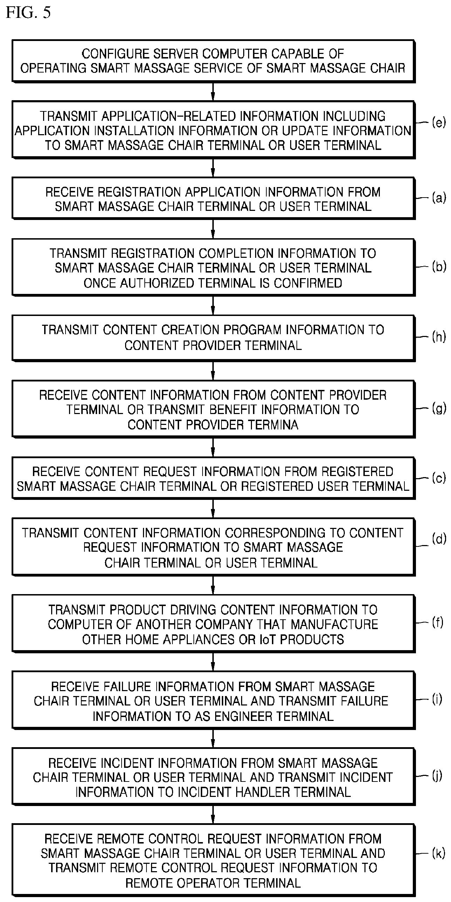

FIG. 5 is a conceptual diagram illustrating a method of operating a smart massage service of a smart massage chair according to some other embodiments of the present invention.

As shown in FIGS. 1 to 5, when sequentially showing the method of operating a smart massage service of a smart massage chair according to some other embodiments of the present invention, the server computer 60 capable of providing a smart massage service of a smart massage chair is first configured, and the method of operating a smart massage service of a smart massage chair may include the steps of: (e) transmitting application-related information including application installation information or update information to the smart massage chair terminal 10 or the user terminal 20 through the network 50 or an application store by using the application information transmission program 67; (a) receiving registration application information from the smart massage chair terminal 10 or the user terminal 20 through the registration application information input program 62; (b) transmitting registration completion information to the smart massage chair terminal 10 or the user terminal 20 through the registration program 63 once the smart massage chair terminal 10 of a company of interest or an authorized massage chair terminal of another company is confirmed by using the registration application information; (h) transmitting content creation program information to the content provider terminal 120 through the content creation program information transmission program 70; (g) receiving the content information from the content provider terminal 120 or transmitting benefit information to the content provider terminal 120 through the content information input program 69; (c) receiving content request information from the registered smart massage chair terminal 10 or the registered user terminal 20 through the content request information input program 65; (d) transmitting content information corresponding to the content request information to the smart massage chair terminal 10 or the user terminal 20 through the content information transmission program 66; (f) transmitting, through the driving program of another company's product, the driving content information of another company's product to the computer 110 of another company that manufactures other home appliances or IoT products; (i) receiving failure information from the smart massage chair terminal 10 or the user terminal 20 and transmitting the failure information to the AS engineer terminal 130 through the failure handling program 72; (j) receiving incident information from the smart massage chair terminal 10 or the user terminal 20 and transmitting the incident information to the incident handler terminal 140 through the incident handling program 73; and (k) receiving remote control request information from the smart massage chair terminal 10 or the user terminal 20 and transmitting the remote control request information to the remote operator terminal 150 through the remote control program 74.

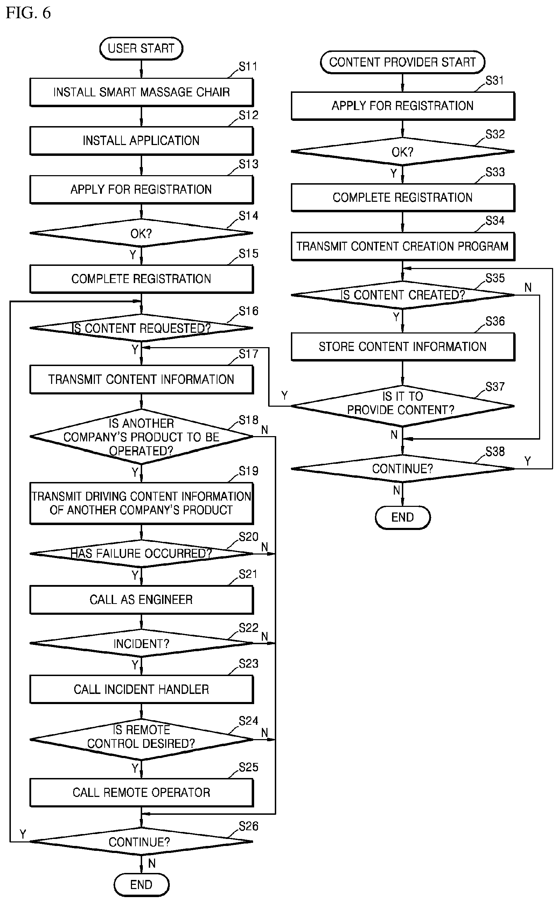

FIG. 6 is a flowchart illustrating a method (process) of operating a smart massage service of a smart massage chair according to still some other embodiments of the present invention.

As shown in FIGS. 1 to 6, the method (process) of operating a smart massage service of a smart massage chair according to some other embodiments of the present invention is described largely from the perspectives of a user (or the first installation technician) and a content provider.

First, from the perspective of the user (or the first installation technician), the user or the first installation technician may install the smart massage chair 1 at an installation location (S 11).

Subsequently, the user or the first installation technician who accesses the network 50, such as the Internet or Wi-Fi, may use the network 50 or an application market to download application-related information including application installation information or update information to the smart massage chair terminal 10 or the user terminal 20 and install an application (S12).

Subsequently, the user or the first installation technician may access the server computer 60 and apply for registration (S13).

Subsequently, when a registration condition input in advance to the server computer 60 is satisfied (S14), the smart massage chair terminal 10 of a company of interest or an authorized massage chair terminal of another company is confirmed by using the registration application information, the registration of the smart massage chair terminal 10 or the user terminal 20 may be completed (S15).

Subsequently, the user may request desired content using the registered smart massage chair terminal 10 or the registered user terminal 20 (S16), and in this case, the server computer 60 may transmit content information corresponding to the content request information to the smart massage chair terminal 10 or the user terminal 20 (S17).

In this case, from the perspective of a content provider who creates the content information, the content provider who desires to create and provide the content may access the server computer 60 and apply for registration (S31). When the registration condition input in advance to the server computer 60 is satisfied (S32), the registration of the content provider terminal 120 may be completed using the registration application information (S33). The server computer 60 may transmit the content creation program information to the registered content provider terminal 120 (S34).

Thereafter, the content provider may create a variety of content information using the content creation program 71 (S35), store the content information in the server computer 60 (S36), and provide the content information to the user (S37). Subsequently, when the content provider wants to continue (S38), the content provider may continue to create and store the content information.

Accordingly, the user may receive the content information provided in this way (S17), and then be provided with various services corresponding to the content information.

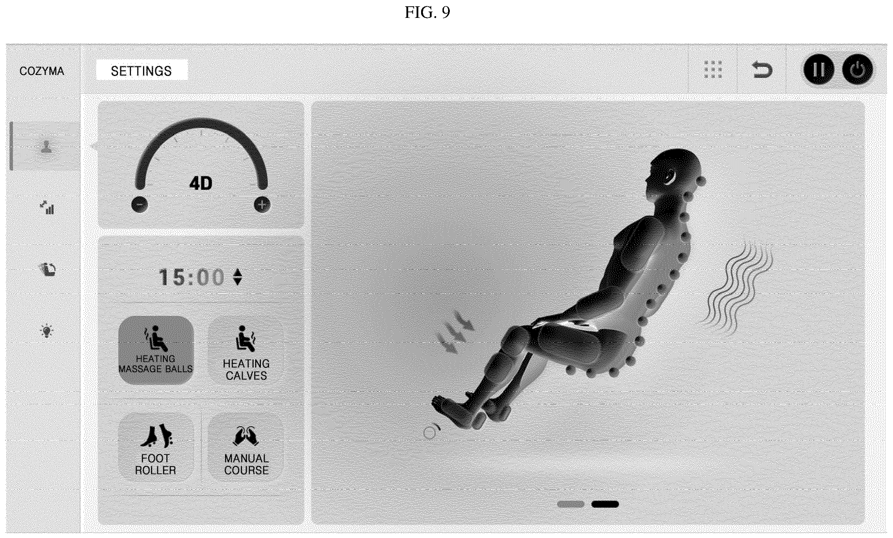

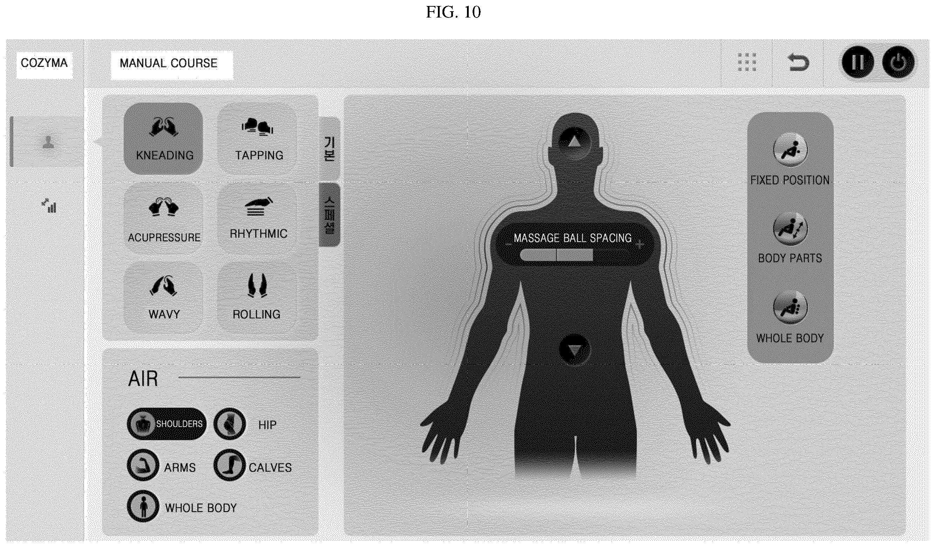

FIGS. 9 and 10 are diagrams illustrating screens provided to terminals of the system of FIG. 1 for operating a smart massage service of a smart massage chair.

The user may be provided with a screen for performing a basic "settings" function on the smart massage chair terminal 10 installed in the smart massage chair 1 or on the user terminal 20, such as a smart pad or a smartphone, as shown in FIG. 9, or may be provided with a screen for controlling a massage pattern, such as a "manual course," as shown in FIG. 10.

For example, when a user wants to operate another electronic product during a massage (S18), the user may operate or control various IoT products, such as a robot cleaner, a television, an air-conditioner, a washing machine, a dryer, and the like, by receiving driving content information of another company's product from the computer 110 of another company that manufactures other home appliances or IoT products.

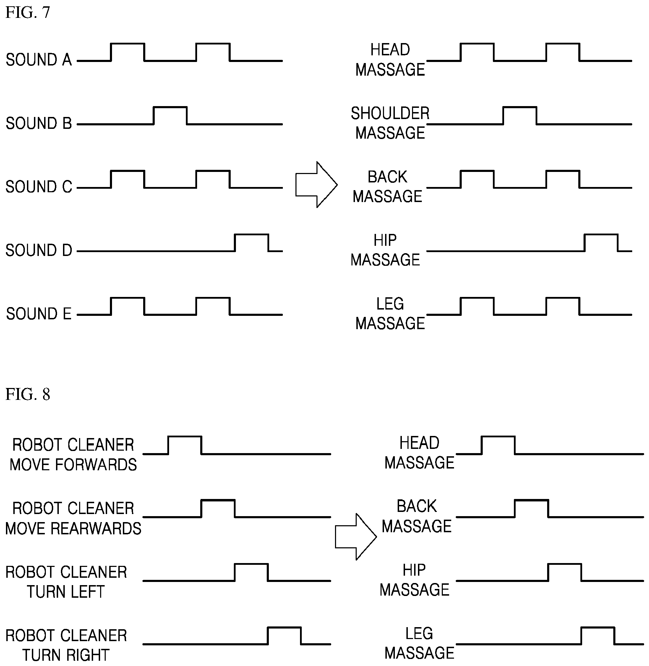

FIGS. 7 and 8 are conceptual diagrams illustrating various examples of content information provided to a user by a method of operating a smart massage service of a smart massage chair according to some embodiments of the present invention.

As shown in FIG. 7, for example, a head massage may be performed while sound A (instrument A) of audio-visual information is being output, a shoulder massage may be performed while sound B (instrument B) of the audio-visual information is being output, a back massage may be performed while sound C (instrument C) of the audio-visual information is being output, a hip massage may be performed while sound D (instrument D) of the audio-visual information is being output, and a leg massage may be performed while sound E (instrument E) of the audio-video information is being output.

Accordingly, the user may receive a massage on various parts of the body at various speeds, intensities, or frequencies in accordance with a variety of audio-visual information, for example, the sound of a musical instrument. This may apply not only to music but also to images. For example, a neck massage may be performed corresponding to the sound of a cannon or an image of a cannon, and a back massage may be performed corresponding to the sound of birds or an image of the sky. However, the present invention is not limited thereto, and the content provider may create content in various forms.

In addition, as shown in FIG. 8, for example, it is possible to drive a robot cleaner of another company during a massage using the smart massage chair terminal 10 or the user terminal 20, and it is also possible to make the user feel the movement of the robot cleaner with his or her body. For example, a head massage is performed when the robot cleaner moves forwards, a back massage is performed when the robot cleaner moves rearwards, a hip massage is performed when the robot cleaner turns left, and a leg massage is performed when the robot cleaner turns right.

In addition, when a failure occurs in the smart massage chair 1 (S20), the user may receive failure information from the smart massage chair terminal 10 or the user terminal 20 and transmit the failure information to the AS engineer terminal 130 or the customer center terminal to call an AS engineer or a customer center (S21).

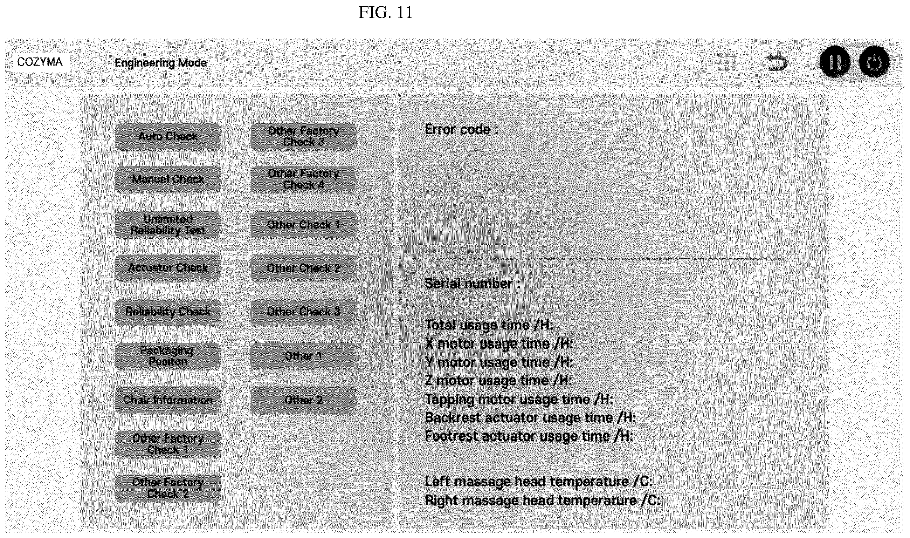

FIGS. 11 to 15 are diagrams illustrating screens provided to terminals of the system of FIG. 1 for operating a smart massage service of a smart massage chair.

As shown in FIG. 11, the AS engineer or the customer center may be provided with a screen showing information on various "check programs" or "error codes" through a terminal, such as a smart pad or a smartphone.

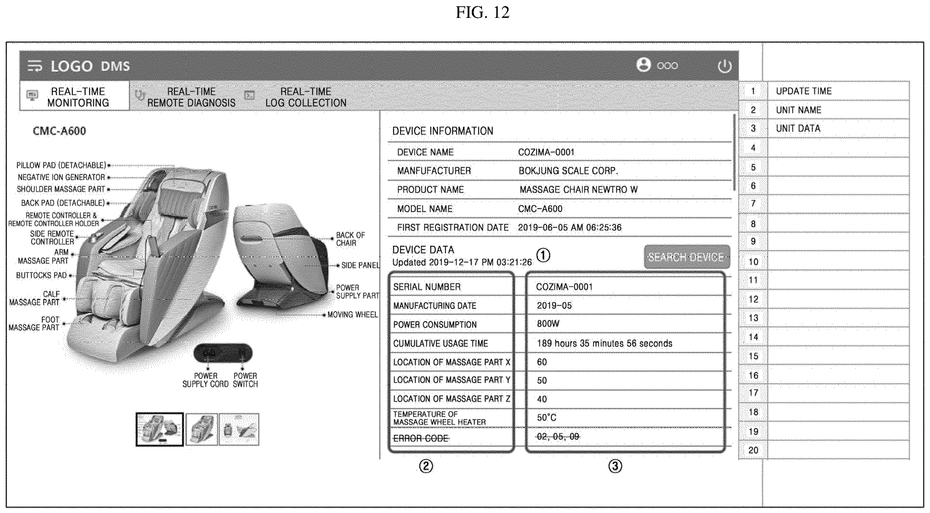

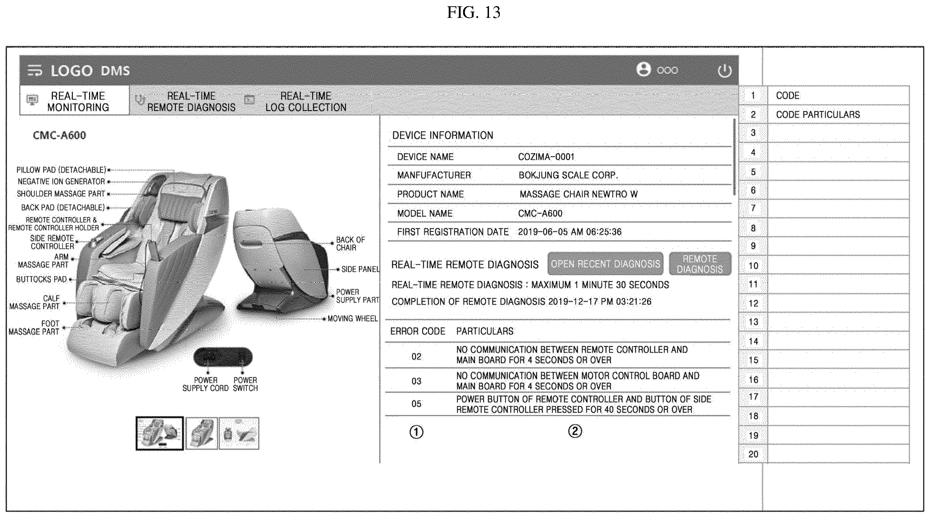

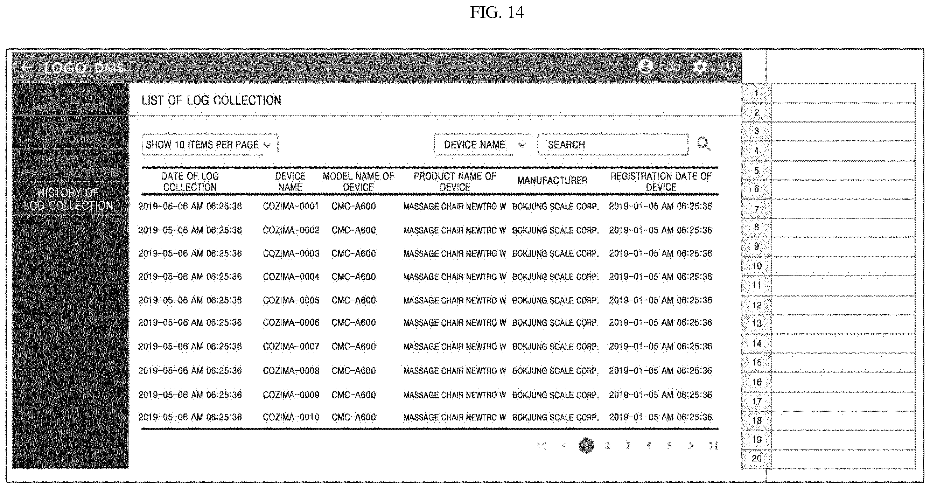

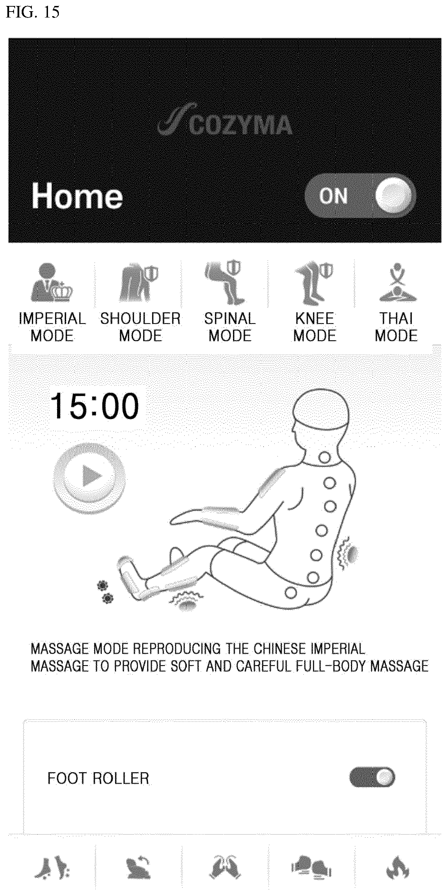

The AS engineer or the customer center may be provided with information on a device name of a product, serial number, error code, and so on as shown in FIG. 12, may check the content of the error code and perform a remote diagnosis in real-time as shown in FIG. 13, and may be provided with a screen showing log history for more accurately determining the cause of the failure as shown in FIG. 14. In addition, as shown in FIG. 15, the smart massage chair 1 may be controlled with the screen of the smartphone. However, these screens are for illustrative purposes only, and are not necessarily limited to the drawings and may be modified and supplemented in a wide variety of forms.

Alternatively, when an incident occurs in the smart massage chair 1 (S22), the user may receive incident information from the smart massage chair terminal 10 or the user terminal 20 and transmit the incident information to the incident handler terminal 140 to call an incident handler (S23).

Alternatively, when the user wants a remote operator, such as a grandchild, a child, an AS engineer, a customer center, or an incident handler, to control his/her smart massage chair 1 (S24), the user may receive remote control request information from the smart massage chair terminal 10 or the user terminal 20 and transmit the remote control request information to the remote operator terminal 150 to call the remote operator (S25).

Subsequently, when the user wants to continue (S38), such content information may be continuously provided.

Meanwhile, the present invention may also be implemented as a computer-readable code on a computer-readable recording medium.

According to various embodiments of the present invention made as described above, a massage chair terminal or a user terminal is connected to the outside through a network or the Internet to allow a user to control the massage order or to be provided with and enjoy various software, coding information, or content information in which various audio-visual information is stored. In addition, users or content providers may create or change and apply various contents at any time after the manufacture of the massage chair or after purchasing the product, and while a user is receiving a massage, the user may perform various other tasks, such as operating another company's product, by linking the massage chair with another company's product, and may also be provided with various smart massage services through organic connection with other users including content providers who desire to create and provide a variety of contents by themselves, AS engineers or customer centers that have to quickly perform AS service, incident handlers, such as on-premise incident handlers, off-premise incident handlers, 911 paramedics, ambulance operators, municipality officers, government officers, or the like, who have to be dispatched in case of emergency, and remote operators who can remotely control the massage chair when a family member or a relative of the user receives the massage, or in case of AS service or emergencies. However, the scope of the present invention is not limited by these effects.

The current embodiments can be implemented as computer readable codes in a computer readable record medium. Codes and code segments constituting the computer program can be easily inferred by a skilled computer programmer in the art. The computer readable record medium includes all types of record media in which computer readable data are stored.

Examples of the computer readable record medium include a ROM, a RAM, a CD-ROM, a magnetic tape, a floppy disk, and an optical data storage. Further, the record medium may be implemented in the form of a carrier wave such as Internet transmission.

In addition, the computer readable record medium may be distributed to computer systems over a network, in which computer readable codes may be stored and executed in a distributed manner.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A method of operating a smart massage service of a smart massage chair by using a computer system including at least one smart massage chair terminal, a user terminal, and a server computer connected through a network, the method comprising:
receiving registration application information from the smart massage chair terminal or the user terminal;
transmitting registration completion information to the smart massage chair terminal or the user terminal once a smart massage chair terminal of a company of interest or an authorized massage chair terminal of another company is confirmed by using the registration application information;
receiving content request information from the registered smart massage chair terminal or the registered user terminal; and
transmitting content information corresponding to the content request information to the smart massage chair terminal or the user terminal.

2. A method of operating a smart massage service of a smart massage chair using a computer system including at least one smart massage chair terminal, a user terminal, and a server computer connected through a network,
wherein the server computer comprises a registration application information input program for receiving registration application information from the smart massage chair terminal or the user terminal, a registration program for transmitting registration completion information to the smart massage chair terminal or the user terminal once a smart massage chair terminal of a company of interest or an authorized massage chair terminal of another company is confirmed by using the registration application information, a content request information input program for receiving content request information from the registered smart massage chair terminal or the registered user terminal, a content information transmission program for transmitting content information corresponding to the content request information to the smart massage chair terminal or the user terminal, a registration application information database in which the registration application information is stored, and a content information database in which the content information is stored and
the method comprises the steps of:
(a) receiving the registration application information from the smart massage chair terminal or the user terminal through the registration application information input program;
(b) transmitting the registration completion information to the smart massage chair terminal or the user terminal through the registration program once a smart massage chair terminal of a company of interest or an authorized massage chair terminal of another company is confirmed by using the registration application information;
(c) receiving content request information from the registered smart massage chair terminal or the registered user terminal through the content request information input program; and
(d) transmitting content information corresponding to the content request information to the smart massage chair terminal or the user terminal through the content information transmission program.

3. The method of claim 2, wherein the registration application information is formed by selecting at least one of model name information, unique information, and serial number information of a smart massage chair, user or purchaser's unique information, other company's massage chair information, input information of an installation technician, an after-sales (AS) engineer, or a customer center, barcode information, quick response (QR) code information, identification code information, radio frequency (RF) signal information, security information, authentication information, and combinations thereof.

4. The method of claim 2, wherein the content information is formed by selecting at least one of home appliance driving content information for driving another home appliance or an Internet of Things (IoT) product, home appliance state notification content information for informing a user of a state of another home appliance or an IoT product through a massage function, emergency notification content information for informing the user of an emergency situation through a massage function, tactile massage-based content information for allowing the user to enjoy sound or an image with a massage function, broadcast massage-based content information for allowing the user to enjoy TV broadcasting by associating a massage broadcast with a massage function, simultaneous massage content information for enabling a plurality of massage chairs to perform massage in the same pattern, and combinations thereof.

5. The method of claim 2, wherein the content information is massage-combined content information in which massage drive information for performing a massage by selecting at least one of user's body parts (feet, legs, buttocks, back, hands, shoulders, neck, head, and the like), massage functions (massage start, massage end, massage type, massage intensity, massage speed, massage time, air and heat function, and the like), and combinations thereof is combined with audio-visual information formed by selecting at least one of voice information, sound information, music information, song information, autonomous sensory meridian response (ASMR) information, graphic information, photo information, image information, video information, movie information, broadcast program, augmented reality information, virtual reality information, tactile information, game information, and combinations thereof.

6. The method of claim 5, wherein the massage-combined content information is formed by selecting at least one of sound-instrument-matching content information in which a corresponding sound or a corresponding instrument sound is matched with each massaged body part of the user, image-effect-matching content information in which a corresponding image effect is matched with each massaged body part of the user, game-effect-matching content information in which a corresponding game effect or a corresponding game motion is matched with each massaged body part of the user, and combinations thereof.

7. The method of claim 6, wherein the massage-combined content information is content information associated with another home appliance, in which a corresponding operation of the another home appliance is matched with at least each massaged body part of the user so that the user can check an actual operation state of the another home appliance or an IoT product through the massaged body part, a speaker, or a monitor.

8. The method of claim 6, wherein the massage-combined content information is content information associated with another massage chair which includes driving command information of another massage chair to allow the user to remotely drive a massage part of the another massage chair.

9. The method of claim 2, wherein
the server computer comprises an application information transmission program for transmitting application-related information including application installation information or update information to the smart massage chair terminal or the user terminal through the network or an application store and an application information database in which the application-related information is stored and
the method further comprises the step of, prior to the step of (a), (e) transmitting the application-related information including application installation information or update information to the smart massage chair terminal or the user terminal through the network or the application store by using the application information transmission program.

10. The method of claim 2, wherein
the server computer comprises a driving program of another company's product for transmitting driving content information of another company's product to a computer of another company that manufactures other home appliances or IoT products and a product driving content information in which the driving content information of another company's product is stored and
the method further comprises the step of, after the step of (d), (f) transmitting, through the driving program of another company's product, the driving content information of another company's product to the computer of another company that manufactures other home appliances or IoT products.

11. The method of claim 2, wherein
the server computer comprises a content information input program for receiving the content information from the content provider terminal or transmitting benefit information to the content provider terminal and
the method further comprises the step of, prior to the step of (c), (g) receiving the content information from the content provider terminal or transmitting the benefit information to the content provider terminal through the content information input program.

12. The method of claim 11, wherein
the server computer comprises a content creation program information transmission program for transmitting content creation program information to the content provider terminal and a content creation program information database in which the content creation program information is stored and
the method further comprises the step of, prior to the step of (g), (h) transmitting the content creation program information to the content provider terminal through the content creation program information transmission program.

13. The method of claim 12, wherein the content creation program comprises:
an audio-visual information input program for inputting audio-visual information formed by selecting at least one of voice information, sound information, music information, song information, ASMR information, graphic information, photo information, image information, video information, movie information, a broadcast program, AR information, virtual reality information, tactile information, game information, and combinations thereof;
a massage drive information input program for inputting the massage drive information for performing a massage by selecting at least one of the user's body parts (feet, legs, buttocks, back, hands, shoulders, neck, head, and the like), massage functions (massage start, massage end, massage type, massage intensity, massage speed, massage time, air and heat function, and the like), and combinations thereof corresponding to each characteristic of the audio-visual information; and
a massage-combined content information storage program for storing the massage-combined content information in which the input audio-visual information is combined with the input massage drive information.

14. The method of claim 2, wherein
the server computer comprises a failure handling program for receiving failure information from the smart massage chair terminal or the user terminal and transmitting the failure information to an AS engineer terminal and a failure information database in which the failure information is stored and
the method further comprises the step of, after the step of (b), (i) receiving the failure information from the smart massage chair terminal or the user terminal and transmitting the failure information to the AS engineer terminal through the failure handling program.

15. The method of claim 14, wherein the failure information is formed by selecting at least one of failure location information indicating a part or location where a failure has occurred, failure code information indicating a failure code capable of identifying the type of failure, failure time information indicating a failure occurrence time, failure cause identification information indicating an operation status of a malfunctioning part before or after a certain period of time to enable identification of the cause of the failure, massage notification information for informing of a location of a malfunctioning part through a massage function in the event of a failure, failure history information indicating the failure history, and combinations thereof.

16. The method of claim 2, wherein
the server computer comprises an incident handling program for receiving incident information from the smart massage chair terminal or the user terminal and transmitting the incident information to an incident handler terminal and an incident information database in which the incident information is stored and
the method further comprises the step of, after the step of (b), (j) receiving incident information from the smart massage chair terminal or the user terminal and transmitting the incident information to the incident handler terminal through the incident handling program.

17. The method of claim 2, wherein
the server computer comprises a remote control program for receiving remote control request information from the smart massage chair terminal or the user terminal and transmitting the remote control request information to a remote operator terminal and a remote control request information database in which the remote control request information is stored and
the method further comprises the step of, after the step of (b), (k) receiving the remote control request information from the smart massage chair terminal or the user terminal and transmitting the remote control request information to the remote operator terminal through the remote operation program.

18. A system for operating a smart massage chair and a smart massage service using a computer system including at least one smart massage chair terminal, a user terminal, and a server computer connected through a network,
wherein the server computer comprises a registration application information input program for receiving registration application information from the smart massage chair terminal or the user terminal, a registration program for transmitting registration completion information to the smart massage chair terminal or the user terminal once a smart massage chair terminal of a company of interest or an authorized massage chair terminal of another company is confirmed by using the registration application information, a content request information input program for receiving content request information from the registered smart massage chair terminal or the registered user terminal, a content information transmission program for transmitting content information corresponding to the content request information to the smart massage chair terminal or the user terminal, a registration application information database in which the registration application information is stored, and a content information database in which the content information is stored and
the server computer comprises a control unit that is programmed to receive the registration application information from the smart massage chair terminal or the user terminal through the registration application information input program, transmit the registration completion information to the smart massage chair terminal or the user terminal through the registration program once a smart massage chair terminal of a company of interest or an authorized massage chair terminal of another company is confirmed by using the registration application information, receive content request information from the registered smart massage chair terminal or the registered user terminal through the content request information input program, and transmit content information corresponding to the content request information to the smart massage chair terminal or the user terminal through the content information transmission program.

19. A computer-readable recording medium having recorded thereon a method of operating a smart massage service of a smart massage chair using a computer system including at least one smart massage chair terminal, a user terminal, and a server computer connected through a network,
wherein the server computer comprises a registration application information input program for receiving registration application information from the smart massage chair terminal or the user terminal, a registration program for transmitting registration completion information to the smart massage chair terminal or the user terminal once a smart massage chair terminal of a company of interest or an authorized massage chair terminal of another company is confirmed by using the registration application information, a content request information input program for receiving content request information from the registered smart massage chair terminal or the registered user terminal, a content information transmission program for transmitting content information corresponding to the content request information to the smart massage chair terminal or the user terminal, a registration application information database in which the registration application information is stored, and a content information database in which the content information is stored and
the method comprises the steps of: (a) receiving the registration application information from the smart massage chair terminal or the user terminal through the registration application information input program; (b) transmitting the registration completion information to the smart massage chair terminal or the user terminal through the registration program once a smart massage chair terminal of a company of interest or an authorized massage chair terminal of another company is confirmed by using the registration application information; (c) receiving content request information from the registered smart massage chair terminal or the registered user terminal through the content request information input program; and (d) transmitting content information corresponding to the content request information to the smart massage chair terminal or the user terminal through the content information transmission program.
